# EUROPÄISCHE PATENTSCHRIFT

(11) **EP 2 381 857 B1**
(45) Veröffentlichungstag und Bekanntmachung des Hinweises auf die Patenterteilung: **17.07.2013**
(21) Anmeldenummer: 09778250.2
(22) Anmeldetag: 01.09.2009
(51) Int. Cl.: A61B 17/16, A61C 1/18

(54) **CHIRURGISCHE ZERSPANVORRICHTUNG**
SURGICAL MACHINING DEVICE
DISPOSITIF DE CISAILLEMENT CHIRURGICAL

(30) Priorität: 30.10.2008 DE 102008053842
(43) Veröffentlichungstag der Anmeldung: 02.11.2011
(73) Patentinhaber: Kirchner, Hilmar O., 5020 Salzburg (AT)
(72) Erfinder: Kirchner, Hilmar O., 5020 Salzburg (AT)
(74) Vertreter: Held, Stephan
(86) Internationale Anmeldenummer: PCT/EP2009/006324
(87) Internationale Veröffentlichungsnummer: WO 2010/049031

(56) Entgegenhaltungen:
- WO-A-2007/137961
- US-A1- 2007 260 227

## Beschreibung

Die Erfindung betrifft eine chirurgische Zerspanvorrichtung, insbesondere zur Anwendung in der Oralchirurgie.

Chirurgische Zerspanvorrichtungen, wie beispielsweise Bohrer oder Fräser, werden vor allem zum Durchtrennen von hartem Knochengewebe eingesetzt. Bekannte Bohrer bzw. Bohrköpfe umfassen einen Schaft, der auf dem Außenumfang angeordnete Schneideflächen aufweist. Die Schneideflächen sind vorzugsweise spiralförmig um den Schaft angeordnet. Alternativ kann zum Durchtrennen von Knochengewebe auch ein Fräser eingesetzt werden, wobei sich die Schneidflächen vorzugsweise parallel zur Rotationsachse erstrecken.

In der Oralchirurgie werden bekannte Zerspanvorrichtungen beispielsweise bei der sogenannten Sinuslift-Operation eingesetzt. Das Ziel dieser Operation besteht darin, die Knochenschicht des Kieferhöhlenbodens zu verdicken, um dessen Stabilität zu erhöhen. Bei dem bekannten Sinuslift-Verfahren wird nach Freilegen des Operationsgebietes durch Abklappen des Zahnfleischs mit einem bekannten, herkömmlichen chirurgischen Bohrer ein Bohrloch gesetzt. Dabei besteht die Gefahr, dass beim Durchtrennen des Kieferknochens die den Kieferknochen bedeckende, dünne Hautschicht beschädigt wird, wenn der Bohrer den Knochen durchdringt.

Aus der WO 2007/137961 A ist eine chirurgische Zerspanvorrichtung bekannt. Sie weist eine Übertragungswelle zur Verbindung mit einem Antrieb und einem rotierbaren, hohlzylindrischen Zerspanelement auf. Das Zerspanelement umfasst eine axiale Durchgangsbohrung zur Führung eines Betätigungselements. Es ist ein Kupplungselement vorgesehen, das zwischen dem Zerspanelement und der Übertragungswelle gelagert und mit dem Betätigungselement und dem Zerspanelement drehfest verbunden ist. Weiterhin wird eine Druckfeder beschrieben, die wirkverbunden mit dem Betätigungselement und mit der Übertragungswelle ist.

Die bekannten Zerspanvorrichtungen bringen also den Nachteil mit, die Verletzungs-gefahr für die dünne Knochenhaut, die sogenannte Schneider'sche Membran, zu erhöhen. Um dieses Risiko zu reduzieren, ist es üblich, den Kieferknochen nicht vollständig zu durchbohren, sondern den Bohrvorgang kurz vor dem Durchbruch zu stoppen. Das Durchbrechen des angebohrten Kieferknochens erfolgt durch einen Stößel, der mit leichten Hammerschlägen vorangetrieben wird. Dieses Verfahren erfordert eine hohe Präzision des behandelnden Kieferchirurgen und einen relativ hohen Zeitaufwand.

Die Aufgabe der Erfindung besteht also darin, eine chirurgische Zerspanvorrichtung anzugeben, die ein einfaches und schnelles Durchtrennen von Knochengewebe ermöglicht, wobei eine Verletzung von Weichteilgewebe vermieden bzw. verhindert wird.

Erfindungsgemäß wird diese Aufgabe durch den Gegenstand des Patentanspruchs 1 gelöst.

Die Erfindung basiert demnach auf dem Gedanken, eine chirurgische Zerspanvorrichtung mit einer Übertragungswelle zur Verbindung mit einem Antrieb und einem rotierbaren, hohlzylindrischen Zerspanelement anzugeben, das eine axiale Durchgangsbohrung zur Führung eines Betätigungselements umfasst, wobei ein Kupplungselement vorgesehen ist, das zwischen dem Zerspanelement und der Übertragungswelle axial verschiebbar gelagert und mit dem Betätigungselement und dem Zerspanelement drehfest verbunden ist. Zwischen dem Kupplungselement und der Übertragungswelle ist eine Druckfeder angeordnet. Ferner ist das Kupplungselement mit dem Betätigungselement wirkverbunden derart, dass eine auf das Betätigungselement wirkende Axialkraft, die gegen die Druckfeder wirkt, das Kupplungselement mit der Übertragungswelle reib- und/oder kraftschlüssig verbindbar ist.

Die erfindungsgemäße, chirurgische Zerspanvorrichtung weist somit einen Kupplungsmechanismus auf, der durch eine axial wirkende Kraft betätigbar ist. Durch den Kupplungsmechanismus wird sichergestellt, dass der Zerspanvorgang, beispielsweise das Bohren oder Fräsen, gestoppt wird, sobald die gegen die Druckfeder wirkende Axialkraft reduziert wird. Solange die Axialkraft entgegen der Druckfeder bzw. in Richtung der Übertragungswelle wirkt, rotiert das Zerspanelement. Sobald das Betätigungselement das Knochengewebe durchdrungen hat und die Axialkraft nachlässt, wird das Kupplungselement durch die Druckfeder in axialer Richtung verschoben, so dass die reib-und/oder kraftschlüssige Verbindung zwischen Kupplungselement und Übertragungswelle gelöst wird. Durch die reib- bzw. kraftschlüssige Verbindung zwischen dem Kupplungselement und der Übertragungswelle wird außerdem erreicht, dass die Rotation des Zerspanelements stoppt, wenn sich das Zerspanelement verhakt.

Der Kupplungsmechanismus der erfindungsgemäßen chirurgischen Zerspanvorrichtung erfüllt also zwei Funktionen. Einerseits verhindert die Kupplung, dass nach Durchtrennen des Knochengewebes dahinter liegendes Weichgewebe, beispielsweise die Schneider'sche Membran, beschädigt bzw. verletzt wird und andererseits eine Verletzung des Knochengewebes erfolgt, wenn das Zerspanelement verhakt. Somit wird mit der Erfindung die Möglichkeit geschaffen, mit einfachen konstruktiven Mitteln eine drehmomentbegrenzte Zerspanvorrichtung bereitzustellen.

Das Betätigungselement ist in einer axialen Durchgangsbohrung des Zerspanelements geführt und steht vorzugsweise über das Zerspanelement hervor, so dass der Zerspanvorgang stoppt, bevor das Zerspanelement das Knochengewebe vollständig durchdrungen hat. Damit wird die Verletzungsgefahr für angrenzendes Weichteilgewebe weiter reduziert.

Das Kupplungselement ist mit dem Betätigungselement wirkverbunden. Die Wirkverbindung ist vorzugsweise dadurch hergestellt, dass das Betätigungselement einen Anschlag aufweist, der an einer Stirnfläche des Kupplungselements anliegt. Durch den Anschlag wird erreicht, dass eine axiale Bewegung bzw. Verschiebung des Betätigungselements, insbesondere in Richtung der Übertragungswelle, eine Verschiebung des Kupplungselements bewirkt. Durch die Verschiebung des Betätigungselements in Richtung der Übertragungswelle wird der Reib- bzw. Kraftschluss zwischen Kupplungselement und Übertragungswelle hergestellt, so dass das durch die Übertragungswelle vom Antrieb aufgenommene Drehmoment an das Zerspanelement übertragen wird, das mit dem Betätigungselement und dem Kupplungselement drehfest verbunden ist.

Bei einer bevorzugten Ausführungsform weist das Zerspanelement an einem axialen Ende einen Eingriffsabschnitt auf, der mit einem Verbindungsabschnitt des Kupplungselements formschlüssig verbunden oder verbindbar ist. Durch den Formschluss wird eine sichere und im Wesentlichen verlustarme Drehmomentübertragung zwischen Kupplungselement und Zerspanelement gewährleistet.

Der Verbindungsabschnitt und der Eingriffsabschnitt können jeweils eine Kontaktfläche umfassen, die im Wesentlichen parallel zu einer Rotationsachse des Zerspanelements ausgerichtet und aneinander anliegend angeordnet sind. Die komplementär zueinander, gegenüber angeordneten Kontaktflächen stellen einerseits eine zuverlässige Drehmomentübertragung sicher und ermöglichen andererseits, dass das Kupplungselement in axialer Richtung verschiebbar ist, wobei ein ständiger Eingriff des Eingriffsabschnitts in den Verbindungsabschnitt gewährleistet ist.

Vorzugsweise weist der Eingriffsabschnitt des Zerspanelements eine axiale Längserstreckung auf, die größer als der maximale Abstand zwischen dem Kupplungselement und der Übertragungswelle ist. Die Kontaktflächen des Verbindungsabschnitts bzw. des Eingriffsabschnitts können aufeinander gleiten, wenn das Kupplungselement axial verschoben wird. Die axiale Verschiebbarkeit des Kupplungselements wird dabei in der einen Richtung durch den Eingriffsabschnitt des Zerspanelements und in der anderen Richtung durch die Übertragungswelle begrenzt. Dadurch, dass die axiale Längserstreckung des Eingriffsabschnitts bzw. der Kontaktfläche des Eingriffsabschnitts größer ist als der maximale Abstand zwischen Kupplungselement und Übertragungswelle, d.h. größer ist als die maximale Verschiebbarkeit des Kupplungselements, ist gewährleistet, dass das Kupplungselement mit dem Zerspanelement im dauerhaften Eingriff ist.

Bei einer weiteren bevorzugten Ausführungsform weist die Übertragungswelle an einem axialen Ende eine Übertragungsfläche auf, die in einem eingekuppelten Zustand mit einer Kupplungsfläche des Kupplungselements reib- und/oder kraftschlüssig verbunden ist. Vorzugsweise sind die Übertragungsfläche und die Kupplungsfläche senkrecht zur Rotationsachse der Übertragungswelle bzw. des Kupplungselements angeordnet. Die Übertragungsfläche und die Kupplungsfläche können im Wesentlichen komplementär ausgebildet sein, so dass im eingekuppelten Zustand eine relativ hohe Flächenpressung erreicht wird. Dadurch wird sichergestellt, dass ein ausreichend hohes Drehmoment übertragen wird, um die zerspanende Bearbeitung von Knochengewebe zu ermöglichen.

Die Druckfeder und das Kupplungselement können in einer Aufnahmehülse angeordnet sein, die mit dem Zerspanelement drehfest verbunden ist. Auf diese Weise sind die mechanischen Bauteile des Kupplungsmechanismus geschützt, so dass die ordnungsgemäße Funktion der Kupplung sichergestellt ist.

Die Aufnahmehülse kann den Eingriffsabschnitt des Zerspanelements und die Übertragungsfläche der Übertragungswelle übergreifen. Der Kupplungsmechanismus ist somit vollständig eingekapselt, wodurch einerseits eine sichere Funktion gewährleistet wird und andererseits eine einfache und hygienische Reinigung der Zerspanvorrichtung ermöglicht wird.

Bevorzugte Materialien für die Zerspanvorrichtung, insbesondere das Betätigungselement, das Zerspanelement und/oder die Aufnahmehülse, sind Chirurgiestahl oder Zirkonkeramik. Derartige Materialien ermöglichen eine besonders einfache und hygienische Reinigung, insbesondere Sterilisation, der Zerspanvorrichtung und bieten eine ausreichende Stabilität zur zerspanenden Bearbeitung von Knochengewebe.

Die Erfindung wird im Folgenden anhand von Ausführungsbeispielen unter Bezugnahme auf die beigefügten, schematischen Zeichnungen näher erläutert. Darin zeigen
- Fig. 1: eine perspektivische Ansicht einer erfindungsgemäßen Zerspanvorrichtung gemäß einem bevorzugten Ausführungsbeispiel;
- Fig. 2: eine Draufsicht auf eine Zerspanvorrichtung gemäß Figur 1;
- Fig. 3: einen Längsschnitt durch eine Zerspanvorrichtung entlang der Linie A-A gemäß Figur 2; und
- Fig. 4: eine perspektivische Explosionsdarstellung der Zerspanvorrichtung gemäß den Figuren 1 bis 3.

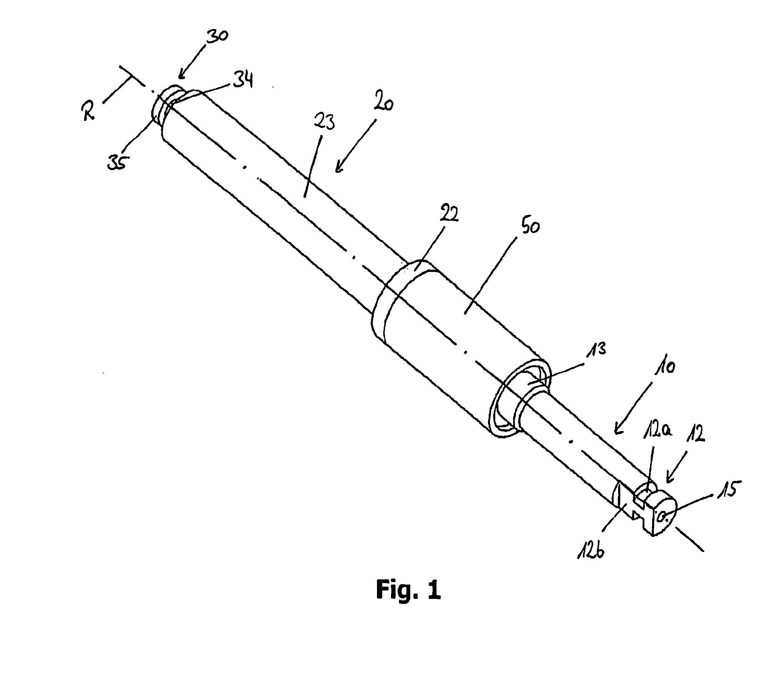

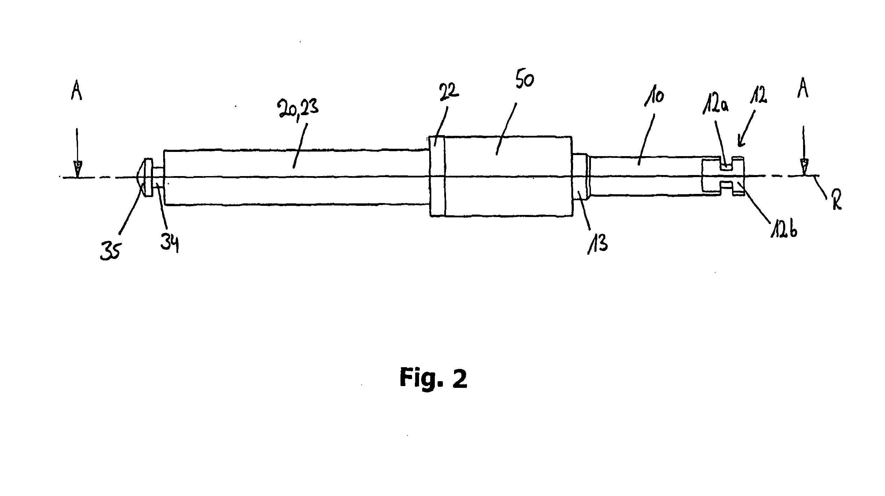

Die Figuren 1 bis 4 zeigen eine chirurgische Zerspanvorrichtung bzw. einen Zerspanaufsatz für ein chirurgisches Instrument gemäß einem bevorzugten Ausführungsbeispiel. Eine derartige Zerspanvorrichtung kann beispielsweise ein Fräser bzw. Fräsaufsatz oder ein Bohrer bzw. Bohraufsatz sein, wobei die Erfindung nicht darauf eingeschränkt ist.

Die Zerspanvorrichtung umfasst eine Übertragungswelle 10, eine Aufnahmehülse 50, ein Zerspanelement 20 und ein Betätigungselement 30. Die Übertragungswelle 10, die Aufnahmehülse 50, das Zerspanelement 20 und das Betätigungselement 30 sind koaxial auf einer gemeinsamen Rotationsachse R angeordnet.

Die Übertragungswelle 10 weist vorzugsweise einen Querschnittsdurchmesser von 1 mm bis 5 mm, insbesondere 2 mm bis 3 mm, insbesondere 2,35 mm, und/oder eine Länge von 8 mm bis 15 mm, insbesondere 10 mm bis 13 mm, insbesondere 11,6 mm, auf. Der äußere Querschnittsdurchmesser der Aufnahmehülse 50 beträgt vorzugsweise zwischen 3 mm und 7 mm, insbesondere zwischen 4 mm und 6 mm, insbesondere 4,8 mm. Die Aufnahmehülse 50 kann sich über eine Länge von 8 mm bis 13 mm, insbesondere 9 mm bis 11 mm, insbesondere 9,6 mm, erstrecken. Die gesamte Zerspanvorrichtung weist vorzugsweise eine Länge von 35 mm bis 50 mm, insbesondere 38 mm bis 45 mm, insbesondere 41 mm, auf.

Die Übertragungswelle 10 ist im Wesentlichen zylinderförmig ausgebildet und weist an einem freien, axialen Ende ein Anschlussstück 12 auf, das eine Ringnut 12a und eine Anschlussfläche 12b umfasst. Die Anschlussfläche 12b ist im Wesentlichen parallel zur Rotationsachse R ausgerichtet, so dass die Übertragungswelle 10 im Bereich des Anschlussstücks 12 einen Teilkreisquerschnitt mit einer flachen bzw. geradlinigen Basis bildet. Die Ringnut 12a schneidet die Anschlussfläche 12b bzw. ist durch die Anschlussfläche 12b unterbrochen. Das Anschlussstück 12 ermöglicht die Verbindung der Zerspanvorrichtung mit einem Antrieb, beispielsweise einem elektromechanischen Bohrer für die Oralchirurgie. Die Zerspanvorrichtung kann auch mit anderen, medizinischen Antrieben verwendet werden, wobei es möglich ist, dass die Übertragungswelle 10 ein andersartig gestaltetes Anschlussstück 12 aufweist.

An dem gegenüber dem Anschlussstück 12 angeordneten axialen Ende der Übertragungswelle 10 weist die Übertragungswelle 10 eine Aufdickung 13 auf. Die Aufdickung 13 ist in einem Innenring 51 der Aufnahmehülse 50 drehbar angeordnet, wobei der Innenring 51 mit der Aufnahmehülse 50 einteilig ausgebildet ist. Der Innenring 51 ist an einem der Übertragungswelle 10 zugewandten axialen Ende der Aufnahmehülse 50 angeordnet. Der Innenring 51 ist in axialer Richtung nach Innen versetzt angeordnet, so dass die Aufnahmehülse 50 an dem der Übertragungswelle 10 zugewandten axialen Ende einen Absatz 52 bildet. Die Aufnahmehülse 50 ist im Allgemeinen hohlzylinderförmig ausgebildet. Innerhalb der Aufnahmehülse 50 ist ein Kupplungsmechanismus (in Figur 1 nicht dargestellt) angeordnet, der später näher erläutert wird.

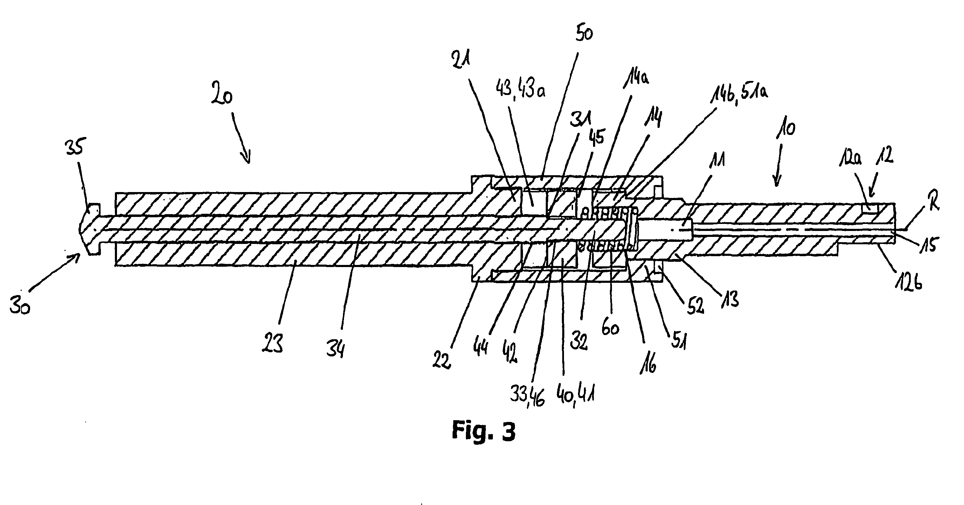

Wie in Figur 3 zu erkennen, weist die Übertragungswelle 10 einen Kanal 15 auf, der die Übertragungswelle 10 im Wesentlichen vollständig in Längsrichtung durchdringt. Der Kanal 15 geht im Bereich der Aufdickung 13 in eine Domaufnahme 11 über, die im Wesentlichen einen größeren Querschnittsdurchmesser aufweist wie der Kanal 15. Der Querschnittsdurchmesser der Dornaufnahme 11 ist derart angepasst, dass ein Dorn 32, der durch ein axiales Ende des Betätigungselements 30 gebildet ist, in die Domaufnahme 11 einführbar ist. Im Inneren der Aufnahmehülse 50 bildet die Übertragungswelle 10 ferner einen Übertragungsabschnitt 14, der einen größeren Querschnittsdurchmesser aufweist als die Aufdickung 13 und der Innendurchmesser des Innenrings 51 der Aufnahmehülse 50. Der Übertragungsabschnitt 14 weist eine Gleitfläche 14b, die sich im Wesentlichen senkrecht zur Rotationsachse R erstreckt und einer Ringfläche 51a des Innenrings 51 gegenüber angeordnet ist.

Im Bereich des Übertragungsabschnitts 14 weist die Übertragungswelle 10 ferner eine Federaufnahme 16 auf, die koaxial zur Domaufnahme 11 bzw. zum Kanal 15 angeordnet ist. Die Federaufnahme 16 weist einen größeren Querschnittsdurchmesser auf als die Dornaufnahme 11. Innerhalb der Federaufnahme 16 ist eine Druckfeder 60 angeordnet, die sich einerseits im Bereich der Aufdickung 13 an der Übertragungswelle 10 und andererseits an einer Kupplungsfläche 45 eines Kupplungselements 40 abstützt. Das Kupplungselement 40 ist im Wesentlichen koaxial zur Übertragungswelle 10 angeordnet. Im ausgekuppelten Zustand, wie in Figur 3 dargestellt, ist das Kupplungselement 40 von der Übertragungswelle 10 beabstandet angeordnet.

Der maximale Abstand des Kupplungselements 40 bzw. der Kupplungsfläche 45 von der Übertragungswelle 10 bzw. der Übertragungsfläche 14a im ausgekuppelten Zustand beträgt vorzugsweise höchstens 1,2 mm, insbesondere höchstens 1 mm, insbesondere höchstens 0,8 mm, insbesondere höchstens 0,6 mm.

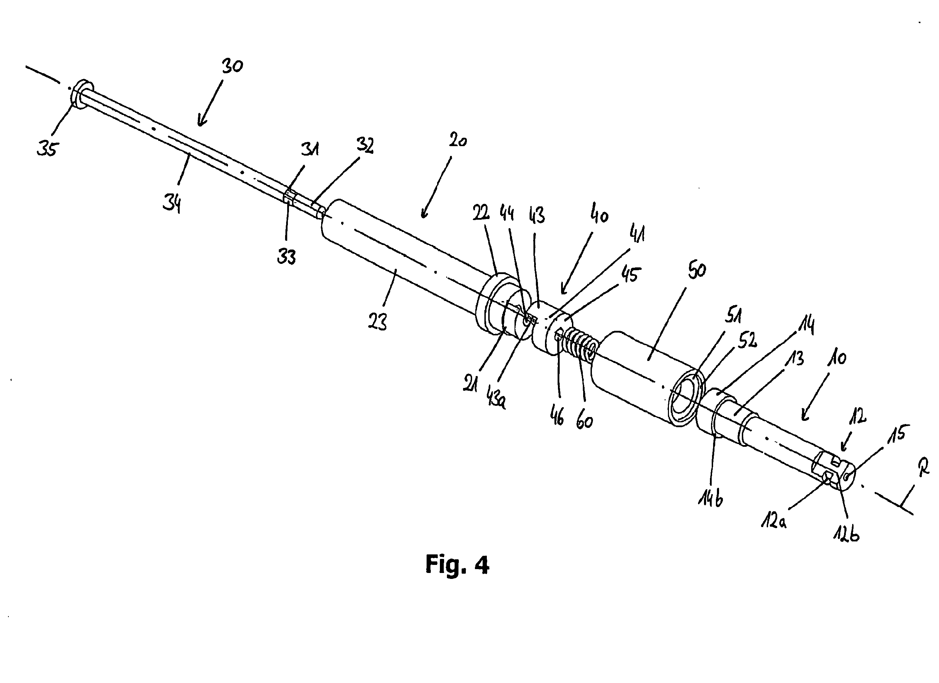

Wie in Figur 4 zu erkennen, weist das Kupplungselement 40 einen im Wesentlichen zylinderförmigen bzw. scheibenartigen Kupplungsabschnitt 41 und einen Verbindungsabschnitt 43 auf, wobei der Verbindungsabschnitt 43 durch einen Fortsatz des hälftigen Kupplungsabschnitts 41 gebildet ist. Der Kupplungsabschnitt 41 weist auf einer der Übertragungswelle 10 zugewandten Seite eine Kupplungsfläche 45 auf, die der Übertragungsfläche 14a gegenüber angeordnet ist. Im eingekuppelten Zustand liegt die Kupplungsfläche 45 an der Übertragungsfläche 14a an und bildet eine reib- bzw. kraftschlüssige Verbindung zwischen dem Kupplungselement 40 und der Übertragungswelle 10.

Im Bereich des Kupplungsabschnitts 41 weist das Kupplungselement 40 ferner eine Mitnahmeöffnung 46 auf, die gemäß dem hier dargestellten Ausführungsbeispiel einen sechskantförmigen Querschnitt umfasst. Andere Querschnittsformen sind möglich. Die Mitnahmeöffnung 46 ist im Wesentlichen komplementär zu einem Mitnahmeabschnitt 33 des Betätigungselements 30 ausgebildet. Bei dem hier dargestellten Ausführungsbeispiel weist der Mitnahmeabschnitt 33 des Betätigungselements 30 ebenfalls eine Sechskantform auf, wobei auch andere Querschnittsprofile möglich sind. Der Mitnahmeabschnitt 33 greift in die Mitnahmeöffnung 46 ein, so dass das Kupplungselement 40 mit dem Betätigungselement 30 drehfest verbunden ist. An den Mitnahmeabschnitt 33 des Betätigungselements 30 schließt sich der Dorn 32 an, der in die Druckfeder 60 hineinragt.

Gegenüber der Kupplungsfläche 45 weist das Kupplungselement 40 im Bereich des Verbindungsabschnitts 43 eine Stirnfläche 42 auf, an der eine Anschlagfläche 31 des Betätigungselements 30 bündig anliegt. Somit besteht zwischen dem Kupplungselement 40 und dem Betätigungselement 30 in axialer Richtung eine kraftschlüssige Verbindung. Die Mitnahmeöffnung 46 ist auf den Kupplungsabschnitt 41 begrenzt, d.h. im Bereich des Verbindungsabschnitts 43 besteht zwischen dem Kupplungselement 40 und dem Betätigungselement 30 keine formschlüssige Verbindung. Vielmehr ist im Verbindungsabschnitt 43 eine Freilaufführung 44 angeordnet, in der das Betätigungselement 30 bzw. ein innerhalb des Zerspanelements 20 geführter Schaft 34 des Betätigungselements 30 frei beweglich geführt ist. Der Verbindungsabschnitt 43 weist ferner zwei Kontaktflächen 43a auf, die im Wesentlichen parallel zur Rotationsachse R der Zerspanvorrichtung angeordnet sind. Den Kontaktflächen des Verbindungsabschnitts 43 liegen komplementäre Kontaktflächen (nicht dargestellt) eines Eingriffsabschnitts 21 des Zerspanelements 20 gegenüber. Die Kontaktflächen 43a bilden mit den komplementären Kontaktflächen des Eingriffsabschnitts 21 eine formschlüssige und drehfeste Verbindung zwischen dem Kupplungselement 40 und dem Zerspanelement 20. Die Kontaktflächen 43a können dabei auf den Kontaktflächen des Eingriffsabschnitts 21 bzw. des Zerspanelements 20 gleiten, so dass eine axiale Verschiebbarkeit des Kupplungselements 40 gewährleistet ist, ohne dass der Formschluss zwischen Kupplungselement 40 und Zerspanelement 20 gelöst wird. Die Längserstreckung, d.h. parallel zur Rotationsachse R, des Eingriffsabschnitts 21 und des Verbindungsabschnitts 43 ist daher größer als der maximale Abstand der Kupplungsfläche 45 des Kupplungselements 40 von der Übertragungsfläche 14a der Übertragungswelle 10.

Das Zerspanelement 20 ist koaxial zur Aufnahmehülse 50 angeordnet und mit der Aufnahmehülse 50 fest, insbesondere drehfest, verbunden. Das Zerspanelement 20 umfasst einen Außenring 22, der im Wesentlichen denselben Umfang aufweist wie die Aufnahmehülse 50. Die äußere Umfangsfläche des Außenrings 22 fluchtet also mit der äußeren Umfangsfläche der Aufnahmehülse 50. Das Zerspanelement 20 umfasst ferner einen zylinderförmigen Schneidabschnitt 23, der sich in axialer Richtung zur gemeinsamen Rotationsachse R erstreckt und an dem der Aufnahmehülse 50 zugewandten axialen Ende durch den Außenring 22 begrenzt ist. Anstelle der zylindrischen Form kann der Schneidabschnitt 23 auch andere Formen umfassen, beispielsweise Kegelformen. Der Schneidabschnitt 23 weist einen kleineren Querschnittsdurchmesser auf als der Außenring 22. Der Schneidabschnitt 23 kann ein kreisförmiges bzw. ringförmiges Querschnittsprofil bilden. Andere Querschnittsprofile sind möglich. Innerhalb der Zerspanelements 20 ist das Betätigungselement 30 koaxial angeordnet bzw. geführt. An einem freien, axialen Ende weist das Betätigungselement 30 eine Schneidspitze 35 auf, die über das Zerspanelement 20 bzw. den Schneidabschnitt 23 hervorsteht. Die Schneidspitze 35 kann Schneidflächen oder Schneidkanten aufweisen, so dass die Schneidspitze 35 zur spanenden Materialabtragung, insbesondere zum Vorbohren bzw. Zentrieren, geeignet ist.

Die Schneidspitze 35 umfasst vorzugsweise einen Querschnittsdurchmesser von 1mm bis 5mm, insbesondere 1 mm bis 4 mm, insbesondere 2 mm bis 3 mm, insbesondere 2,3mm. Besonders bevorzugt ist ein Durchmesser der Schneidespitze 35 im Bereich von 3,0mm bis 4,7mm. Beispielsweise kann die Schneidespitze 35 einen Durchmesser von 3,0mm, insbesondere 3,5mm, insbesondere 4,2mm, insbesondere 4,7mm umfassen.

Der zylinderförmige Schneidabschnitt 23 kann einen Querschnittsdurchmesser von 2,5 mm bis 8 mm, insbesondere 3 mm bis 6 mm, insbesondere 3,3 mm, aufweisen. Die Länge des Schneidabschnitts 23 kann zwischen 12 mm und 25 mm, insbesondere zwischen 16 mm und 22 mm, insbesondere 18 mm, betragen.

Im Folgenden wird die Funktionsweise der Zerspanvorrichtung beschrieben:
Wenn die Übertragungswelle 10 bzw. der Aufnahmedorn durch das Anschlussstück 12 mit einem Antrieb bzw. einer Antriebseinheit verbunden ist, wird das Drehmoment des Antriebs in die Übertragungswelle 10 eingeleitet. Im unbelasteten Zustand, d.h. ohne eine Einwirkung einer Axialkraft auf das Betätigungselement 30 bzw. den Fräsdorn in Richtung der Übertragungswelle 10 bzw. dem Aufnahmedorn besteht zwischen dem Zerspanelement 20 bzw. dem Fräser und der Übertragungswelle 10 bzw. dem Aufnahmedorn keine drehmomentübertragende Verbindung. Sobald das Betätigungselement 30 bzw. der Fräsdorn beispielsweise durch Aufsetzen der Zerspanvorrichtung auf hartes Knochengewebe mit einer Axialkraft beaufschlagt wird, wird das Betätigungselement 30 axial in Richtung der Übertragungswelle 10 verschoben. Durch die Anschlagsfläche 31, die an der Stirnfläche 42 des Kupplungselements 40 anliegt, wird das Kupplungselement 40 ebenfalls in axialer Richtung gegen die Übertragungswelle 10 bzw. den Aufnahmedorn verschoben. Dabei gleiten die Kontaktflächen 43a des Verbindungsabschnitts 43 des Kupplungselements 40 auf den gegenüber angeordneten Kontaktflächen (nicht dargestellt) des Eingriffsabschnitts 21 des Zerspanelements 20. Bei diesem Vorgang wird der Kupplungsabschnitt 41 des Kupplungselements 40 zwar vom Eingriffsabschnitt 21 des Zerpanelements 20 getrennt. Durch die Längserstreckung des Verbindungsabschnitts 43 bzw. der Kontaktflächen 43a ist jedoch eine formschlüssige und drehfeste Verbindung zwischen Kupplungselement 40 des Zerspanelements 20 und Zerspanelement 20 bzw. Fräser gewährleistet.

Durch die auf das Betätigungselement 30 aufgebrachte Kraft und die damit bewirkte Verschiebung des Kupplungselements 40 in Richtung der Übertragungswelle 10 bzw. des Aufnahmedorns wird eine reibschlüssige bzw. kraftschlüssige Verbindung zwischen der Kupplungsfläche 45 des Kupplungselements 40 und der Übertragungsfläche 14a der Übertragungswelle 10 bzw. des Aufnahmedorns erreicht. Das in die Übertragungswelle 10 eingeleitete Drehmoment des Antriebs wird durch die reibschlüssige Verbindung an das Kupplungselement 40 übertragen. Die Drehmomentweiterleitung erfolgt über die formschlüssige und drehfeste Verbindung zwischen Kupplungselement 40, Zerspanelement 20 bzw. Fräser und Betätigungselement 30 bzw. Fräsdorn. Da das Zerspanelement 20 bzw. der Fräser mit der Aufnahmehülse 50 ebenfalls drehfest verbunden ist, bewirkt das Einkuppeln eine Rotation aller Bauteile der Zerspanvorrichtung.

Zur Betätigung des Kupplungsmechanismus' ist es zweckmäßig, auf das Betätigungselement 30 bzw. den Fräsdorn eine Kraft aufzubringen, die größer ist als die durch die Druckfeder 60 erzeugte Gegenkraft. Dazu ist die Druckfeder 60 vorzugweise derart dimensioniert, dass für den chirurgischen, insbesondere kieferchirurgischen bzw. minimalchirurgischen, Einsatz ein leichtes Anpressen der Zerspanvorrichtung bzw. des Betätigungselements 30 gegen hartes Knochengewebe ein Einkuppeln bewirkt. Sobald das harte Knochengewebe durch das Betätigungselement 30 bzw. den Fräsdorn durchdrungen ist, lässt die Axialkraft nach, so dass die Federkraft der Druckfeder 60 überwiegt, wodurch das Kupplungselement 40 von der Übertragungswelle 10 getrennt wird. Damit ist der Drehmomentfluss unterbrochen und das Betätigungselement 30, das Zerspanelement 20 und die Aufnahmehülse 50 stoppen die Rotation.

Zusätzlich ermöglicht der Kupplungsmechanismus ein Stoppen des Zerspanvorgangs bzw. der Rotation des Zerspanelements 20, wenn das Betätigungselement 30 bzw. die Schneidspitze 35 in Rotationsrichtung einer erhöhten Gegenkraftkomponente, d.h. einem Drehmoment, das gegen die Rotation des Zerspanelements 20 bzw. Betätigungselements 30 wirkt, ausgesetzt ist. In dem Fall, dass die Schneidspitze 35 im Knochengewebe verhakt, wirkt auf das Betätigungselement 30 bzw. den Fräsdorn eine Gegenkraft zum übertragenen Drehmoment, so dass der Reibschluss zwischen der Kupplungsfläche 45 des Kupplungselements 40 und der Übertragungsfläche 14a der Übertragungswelle 10 unterbrochen wird. Die Übertragungsfläche 14a gleitet dabei auf der Kupplungsfläche 45.

Das bevorzugte Einsatzgebiet der erfindungsgemäßen Zerspanvorrichtung betrifft die Oralchirurgie, wobei die Erfindung nicht darauf einschränkt ist. Es ist auch möglich, die Zerspanvorrichtung in anderen medizinischen Bereichen, beispielsweise der Neurochirurgie, einzusetzen. Besonders bevorzugt ist die Verwendung der Zerspanvorrichtung bei implantologischen Eingriffen im Oberkiefer.

Die Kieferhöhle ist nach innen durch die trennende Knochenwand zur Nasenhöhle, zur Seite nach außen durch die Knochenwand der Wange und der Jochbeinwurzel begrenzt. Nach oben wird die große Kieferhöhle durch eine dünne Knochendecke von der Augenhöhle getrennt, wobei die Knochendecke den Boden der Augenhöhle bildet. Die untere Begrenzung der Kieferhöhle bildet der Sinusboden, der die Zahnwurzeln der kleinen (prämolaren) und großen (molaren) Backenzähne umfasst. Der Sinusboden bildet die knöcherne Trennung der Kieferhöhle von der Mundhöhle.

Es hat sich gezeigt, dass nach Entfernung (Extraktion) eines oder mehrerer Backenzähne die Stärke des Sinusbodens durch Knochenschwund (Atrophie) abnimmt, da die interne Kaukraftbelastung des Knochens reduziert ist. Auch die verringerte Ernährung der Knochenschicht durch die Zähne bewirkt den Knochenschwund, da sich der lebende Zahn in einer gegenseitigen Ernährungsdynamik mit dem ihm tragenden Knochen befindet.

Wenn alle Backenzähne entfernt und auf der Mundseite durch eine auf dem Zahnfleisch aufliegende Zahnprothese ersetzt sind, bewirkt der Kaudruck der Zahnprothese auf den Sinusboden einen zusätzlichen Knochenschwund. Im zeitlichen Verlauf reduziert sich die Knochenschicht des Sinusbodens in vielen Fällen zu einer dünnen Lamelle mit weniger als 1 mm Stärke.

Mit der Sinusliftoperation soll daher erreicht werden, die Knochenschicht des Sinusbodens zu verdicken, wobei bei dieser Operationsmethode die Innenseite der Kieferhöhlenschleimhaut angehoben wird. Nach der Verdickung des Sinusbodens ist es wieder möglich, Implantate in den Kieferknochen einzusetzen.

Bei der Zahnimplantation im Sinuslift-Verfahren unterscheidet man grundsätzlich zwei Varianten, die direkte und die indirekte Sinusliftoperation. Grundsätzlich ist die Sinusliftoperation nach den bekannten Verfahren eine invasive Operationsmethode, die eine relativ hohe Belastung für den Patienten mit sich bringt.

Im Folgenden wird die indirekte Sinusliftoperation im Hinblick auf die Anwendung der erfindungsgemäßen Zerspanvorrichtung erläutert:
Bei dem herkömmlichen Operationsverfahren wird nach Freilegen des Operationsgebietes durch Abklappen des Zahnfleisches mit einem bekannten chirurgischen Bohrer ein Bohrloch für ein zylindrisches Implantatlager bis dicht an die Innenauskleidung der Kieferhöhle vorangebracht und die Innenauskleidung mit einem anderen Instrument, beispielsweise einem Metallstößel bzw. Osteotom, mechanisch mit leichten Hammerschlägen angehoben. Aufgrund der Lautstärke der Hammerschläge wird das Verfahren von dem Patienten als äußerst unangenehm empfunden, so dass die Operation meist unter Vollnarkose durchgeführt wird. Die Vollnarkose stellt für den Patienten ein erhöhtes, gesundheitliches Risiko dar. Ferner besteht eine hohe Verletzungsgefahr der Kieferhöhlenauskleidung (Schneider'sche Membran) durch das Anbohren mit dem herkömmlichen chirurgischen Bohrer bzw. durch die Rissgefahr der Membran bedingt durch die Hammerschläge auf den Metallstößel. Der Zeitaufwand der bekannten Operation beträgt ca. 30 bis 45 Minuten. Das Bohrloch wird im Anschluss mit Fremdmaterial oder Eigenknochen angefüllt, so dass sich mit Einbringen des Implantatkörpers das Material unter der Schneider'schen Membran ausbreiten kann, wodurch der Sinusboden in diesem Bereich verstärkt wird.

Mit der erfindungsgemäßen Zerspanvorrichtung wird hingegen eine minimalinvasive Operation ermöglicht. Ebenfalls nach Freilegen des Operationsgebietes durch Abklappen des Zahnfleisches wird der freiliegende Knochen mit einem herkömmlichen Bohrer angebohrt (ca. 0,5 mm). Anschließend wird die erfindungsgemäße Zerspanvorrichtung eingesetzt, die den äußeren harten Knochen (Compacta), den inneren weichen Knochen (Spongiosa) und die innere Compacta durchbohrt. Sobald die Schneider'sche Membran erreicht ist, stoppt der Fräser bzw. das Zerspanelement 20, wobei der Antrieb weiterhin rotiert. Auf diese Weise wird die Membran, die die Kieferhöhle schützt, nicht verletzt, sondern etwa 3,0 bis 5,0 mm angehoben. Das Bohrloch wird im Anschluss mit Fremdmaterial oder Eigenknochen aufgefüllt, so dass sich mit Einbringen des Implantats das Material unterhalb der Membran der Kieferhöhle ausbreiten kann und keine weitere Verletzungsgefahr für die Schneider'sche Membran besteht. Der Zeitaufwand der Operation bei Verwendung der erfindungsgemäßen Zerspanvorrichtung beträgt ca. 5 Minuten.

Die direkte Sinusliftoperation wird im Vergleich mit der bisher üblichen Methode wie folgt durchgeführt:
Nach Freilegen der seitlichen Kieferhöhlenwand durch Abklappen des Zahnfleisches wird bei dem herkömmlichen Operationsverfahren die seitliche Kieferhöhlenwand in einem ca. 2 cm bis 3 cm großen Bereich durch eine umlaufende Linie durch einen kugelförmigen Diamantbohrer geschwächt, so dass sich die seitliche Kieferhöhlenwand eindrücken lässt. Der Zeitaufwand beträgt ca. 40 bis 60 Minuten. Dabei besteht bei dem herkömmlichen Verfahren die Gefahr, dass der Diamantbohrer die Knochenwand durchbricht und durch den Bohrer die Schneider'sche Membran verletzt wird.

Im weiteren Verlauf der Operation wird der durch den Diamantbohrer erzeugte Deckel zusammen mit der schneiderschen Membran nach innen, oben geklappt, so dass ein Hohlraum entsteht. In diesen Hohlraum werden Knochenchips aus Eigen- oder Fremdknochen oder auch synthetisches Knochenersatzmaterial eingebracht. Das bekannte Verfahren ist für den Patienten schmerzhaft und unangenehm und in der Operationsmethodik aufwändig. Besonders nachteilig sind postoperative Schmerzen und Schwellungen im Operationsbereich. Des Weiteren ist die Wartezeit bis zum Einsetzen eines Implantats relativ lang (ca. 6 Monate).

Mit der erfindungsgemäßen Zerspanvorrichtung wird hingegen eine minimalinvasive Operation ermöglicht. Dabei wird nach Freilegen der seitlichen Kieferhöhlenwand durch Abklappen des Zahnfleisches mit der Zerspanvorrichtung bzw. einem Fräser ein Fenster in die seitliche Kieferhöhlenwand eingebracht (Durchmesser ca. 4,0 bis 30,0 mm). Wenn die Schneider'che Membran erreicht ist, stoppt das Zerspanelement 20 bzw. der Fräskopf (Trepankopf), wobei der Antrieb sowie die Übertragungswelle 10 weiterhin rotiert. Auf diese Weise wird die Membran, die die Kieferhöhle schützt, geschont.

Die Membran wird ca. 0,3 bis 0,5 mm angehoben. Im Anschluss wird das Bohrloch mit Fremdmaterial (Knochenersatzmaterial) oder Eigenknochen angefüllt und das Fenster mit einer Titanmembran oder resorbierbaren Membran abgedeckt. Die Schleimhaut (Gingiva) wird wieder an die Kieferhöhlenwand angelegt und das Operationsgebiet zugenäht. Der Zeitaufwand der Operation mit der erfindungsgemäßen Zerspanvorrichtung beträgt ca. 10 Minuten.

Zusammenfassend besteht die Neuheit der erfindungsgemäßen chirurgischen bzw. minimalchirurgischen Zerspanvorrichtung darin, dass nach der Knochendurchtrennung der Zerspanvorgang sofort und automatisch bei Kontakt mit Gewebe wie Membranen, Weichteilen oder Haut stoppt, so dass derartige Gewebe nicht beschädigt, verletzt oder durchtrennt werden. Damit wird bei dem Einsatz der Zerspanvorrichtung eine verbesserte Wirtschaftlichkeit (Zeiterspamis) erreicht. Medizinische Vorteile betreffen die weitgehende Risikominimierung, die minimalinvasive Operation und die Herabsetzung der postoperativen Auswirkungen für den Patienten.

Die erfindungsgemäße Zerspanvorrichtung kann auch im Bereich der Neurochirurgie eingesetzt werden, wobei mit der Zerspanvorrichtung eine Schädelöffnung ermöglicht wird, ohne die Hirnhaut (Dura Mater) zu beschädigen. Die erfindungsgemäße Zerspanvorrichtung ist allgemein in der minimalinvasiven Knochenchirurgie einsetzbar.

**Bezugszeichenliste**

| | |
|---|---|
| 10 | Übertragungswelle |
| 11 | Dornaufnahme |
| 12 | Anschlussstück |
| 12a | Ringnut |
| 12b | Anschlussfläche |
| 13 | Aufdickung |
| 14 | Übertragungssbschnitt |
| 14a | Übertragungsfläche |
| 14b | Gleitfläche |
| 15 | Kanal |
| 16 | Federaufnahme |
| 20 | Zerspanelement |
| 21 | Eingriffsabschnitt |
| 22 | Außenring |
| 23 | Schneidabschnitt |
| 30 | Betätigungselement |
| 31 | Anschlagfläche |
| 32 | Dorn |
| 33 | Mitnahmeabschnitt |
| 34 | Schaft |
| 35 | Schneidspitze |
| 40 | Kupplungselement |
| 41 | Kupplungsabschnitt |
| 42 | Stirnfläche |
| 43 | Verbindungsabschnitt |
| 43a | Kontaktfläche |
| 44 | Freilaufführung |
| 45 | Kupplungsfläche |
| 46 | Mitnahmeöffnung |
| 50 | Aufnahmehülse |
| 51 | Innenring |
| 51a | Ringfläche |
| 52 | Absatz |
| 60 | Druckfeder |
| R | Rotationsachse |

## Patentansprüche

1. Chirurgische Zerspanvorrichtung mit einer Übertragungswelle (10) zur Verbindung mit einem Antrieb und einem rotierbaren, hohlzylindrischen Zerspanelement (20), das eine axiale Durchgangsbohrung zur Führung eines Betätigungselements (30) umfasst, wobei ein Kupplungselement (40) vorgesehen ist, das zwischen dem Zerspanelement (20) und der Übertragungswelle (10) axial verschieblich gelagert und mit dem Betätigungselement (30) und dem Zerspanelement (20) drehfest verbunden ist, wobei zwischen dem Kupplungselement (40) und der Übertragungswelle (10) eine Druckfeder (60) angeordnet und das Kupplungselement (40) mit dem Betätigungselement (30) wirkverbundbar ist derart, dass durch eine auf das Betätigungselement (30) wirkende Axialkraft, die gegen die Druckfeder (60) wirkt, das Kupplungselement (40) mit der Übertragungswelle (10) reib- und/oder kraftschlüssig verbindbar ist.

2. Chirurgische Zerspanvorrichtung nach Anspruch 1, **dadurch gekennzeichnet, dass** das Betätigungselement (30) eine Anschlagfläche (31) aufweist, die an einer Stirnfläche (42) des Kupplungselements (40) anliegt.

3. Chirurgische Zerspanvorrichtung nach Anspruch 1 oder 2, **dadurch gekennzeichnet, dass** das Zerspanelement (20) an einem axialen Ende einen Eingriffsabschnitt (21) aufweist, der mit einem Verbindungsabschnitt (43) des Kupplungselements (40) formschlüssig verbunden oder verbindbar ist.

4. Chirurgische Zerspanvorrichtung nach Anspruch 3, **dadurch gekennzeichnet, dass** der Verbindungsabschnitt (43) und der Eingriffsabschnitt (21) jeweils wenigstens eine Kontaktfläche (43a) umfassen, die im Wesentlichen parallel zu einer Rotationsachse R des Zerspanelements (20) ausgerichtet und aneinander anliegend angeordnet sind.

5. Chirurgische Zerspanvorrichtung nach Anspruch 4, **dadurch gekennzeichnet, dass** der Eingriffsabschnitt (21) des Zerspanelements (20) eine axiale Längserstreckung aufweist, die größer als der maximale Abstand zwischen dem Kupplungselement (40) und der Übertragungswelle (10) ist.

6. Chirurgische Zerspanvorrichtung nach wenigstens einem der Ansprüche 1 bis 5. **dadurch gekennzeichnet, dass** die Übertragungswelle (10) an einem axialen Ende eine Übertragungsfläche (14a) aufweist, die in einem eingekuppelten Zustand mit einer Kupplungsfläche (45) des Kupplungselements (40) reib-und/oder kraftschlüssig verbunden ist.

7. Chirurgische Zerspanvorrichtung nach wenigstens einem der Ansprüche 1 bis 6, **dadurch gekennzeichnet, dass** die Druckfeder (60) und das Kupplungselement (40) in einer Aufnahmehülse (50) angeordnet sind, die mit dem Zerspanelement (10) drehfest verbunden ist.

8. Chirurgische Zerspanvorrichtung nach Anspruch 7, **dadurch gekennzeichnet, dass** die Aufnahmehülse (50) den Engriffsabschnitt (21) des Zerspanelements (20) und die Übertragungsfläche (14a) der Übertragungswelle (10) übergreift.

## Claims

1. A surgical cutting device, comprising a transmission shaft (10) for connecting to a drive and a rotatable, hollow-cylindrical cutting element (20) having an axial through bore for guiding an actuating element (30), wherein a coupling element (40) is provided which is axially movably supported between said cutting element (20) and said transmission shaft (10) and rotationally fixedly connected to said actuating element (30) and to said cutting element (20), wherein a compression spring (60) is arranged between said coupling element (40) and said transmission shaft (10) and said coupling element (40) is effectively connectable to said actuating element (30), such that said coupling element (40) is frictionally and/or non-positively connectable to said transmission shaft (10) by an axial force acting on said actuating element (30) and acting against said compression spring (60).

2. The surgical cutting device according to claim 1, **characterised in that** said actuating element (30) comprises an abutment surface (31) bearing against an end surface (42) of said coupling element (40).

3. The surgical cutting device according to claim 1 or 2, **characterised in that** said cutting element (20) comprises an engagement portion (21) at an axial end thereof which is positively connected or connectable to a connecting portion (43) of said coupling element (40).

4. The surgical cutting device according to claim 3, **characterised in that** said connecting portion (43) and said engagement portion (21) each comprise a contact surface (43a) which are aligned substantially parallel with a rotational axis R of said cutting element (20) and are arranged in a mutually abutting manner.

5. The surgical cutting device according to claim 4, **characterised in that** said engagement portion (21) of said cutting element (20) has a longitudinal extension which is larger than the maximum distance between said coupling element (40) and said transmission shaft (10).

6. The surgical cutting device according to at least one of the claims 1 to 5, **characterised in that** said transmission shaft (10) comprises a transmission surface (14a) at an axial end thereof which in an engaged condition is frictionally and/or non-positively connected to a coupling surface (45) of said coupling element (40).

7. The surgical cutting device according to at least one of the claims 1 to 6, **characterised in that** said compression spring (60) and said coupling element (40) are arranged in a reception sleeve (50) which is rotationally fixedly connected to said cutting element (10).

8. The surgical cutting device according to claim 7, **characterised in that** said reception sleeve (50) overlaps said engagement portion (21) of said cutting element (20) and said transmission surface (14a) of said transmission shaft (10).

## Revendications

1. Dispositif de coupe chirurgical avec un arbre de transmission (10) pour la liaison avec un entraînement et un élément de coupe (20) cylindrique creux rotatif comportant un alésage axial traversant pour le guidage d'un élément d'actionnement (30), un élément d'accouplement (40) étant prévu, lequel est monté de manière axialement mobile entre l'élément de coupe (20) et l'arbre de transmission (10) et est raccordé de manière solidaire en rotation à l'élément d'actionnement (30) et à l'élément de coupe (20), un ressort de pression (60) étant disposé entre l'élément d'accouplement (40) et l'arbre de transmission (10), et l'élément d'accouplement (40) pouvant être mis en liaison de coopération avec l'élément d'actionnement (30) de telle manière que l'élément d'accouplement (40) peut être raccordé par friction et/ou ou par adhérence à l'arbre de transmission (10) sous l'effet d'un effort axial s'exerçant sur l'élément d'actionnement (30) et agissant contre le ressort de pression (60).

2. Dispositif de coupe chirurgical selon la revendication 1, **caractérisé en ce que** l'élément d'actionnement (30) présente une surface de butée (31) reposant contre une surface frontale (42) de l'élément d'accouplement (40).

3. Dispositif de coupe chirurgical selon la revendication 1 ou 2, **caractérisé en ce que** l'élément de coupe (20) présente une partie d'engagement (21) à une extrémité axiale, laquelle est raccordée ou peut être raccordée par correspondance de forme à une partie de connexion (43) de l'élément d'accouplement (40).

4. Dispositif de coupe chirurgical selon la revendication 3, **caractérisé en ce que** la partie de connexion (43) et la partie d'engagement (21) comprennent chacune au moins une surface de contact (43a), lesdites surfaces de contact étant sensiblement parallèles à un axe de rotation R de l'élément de coupe (20) et adjacentes l'une à l'autre.

5. Dispositif de coupe chirurgical selon la revendication 4, **caractérisé en ce que** la partie d'engagement (21) de l'élément de coupe (20) présente une longueur axiale supérieure à l'espacement maximal entre l'élément d'accouplement (40) et l'arbre de transmission (10).

6. Dispositif de coupe chirurgical selon au moins l'une des revendications 1 à 5, **caractérisé en ce que** l'arbre de transmission (10) présente une surface de transmission (14a) à une extrémité axiale, laquelle est dans un état d'accouplement raccordée par friction et/ou ou par adhérence à une surface d'accouplement (45) de l'élément d'accouplement (40).

7. Dispositif de coupe chirurgical selon au moins l'une des revendications 1 à 6, **caractérisé en ce que** le ressort de pression (60) et l'élément d'accouplement (40) sont logés dans un manchon de réception (50) qui est raccordé de manière solidaire en rotation à l'élément de coupe (10).

8. Dispositif de coupe chirurgical selon la revendication 7, **caractérisé en ce que** le manchon de réception (50) recouvre la partie d'engagement (21) de l'élément de coupe (20) et la surface de transmission (14a) de l'arbre de transmission (10).
